# EUROPEAN PATENT SPECIFICATION

(11) **EP 2 946 738 B1**
(45) Date of publication and mention of the grant of the patent: **12.06.2019**
(21) Application number: 14200060.3
(22) Date of filing: 23.12.2014
(51) Int. Cl.: A61B 18/14, A61B 18/00

(54) **Multipurpose electrosurgical instrument with telescoping aspiration cannula**
Multifunktionales elektrochirurgisches Instrument mit teleskopischer Aspirationskanüle
Instrument électrochirurgical multifonctions à canule d'aspiration télescopique

(30) Priority: 21.05.2014 US 201462001186 P; 08.12.2014 US 201414562947
(43) Date of publication of application: 25.11.2015
(73) Proprietor: Covidien LP, Mansfield, MA 02048 (US)
(72) Inventor: Hufnagel, Elzabeth R., Boulder, Colorado 80302 (US); Trudel, Gregory J., Broomfield, Colorado 80023 (US)
(74) Representative: Soames, Candida Jane

(56) References cited:
- WO-A1-2014/032157
- GB-A- 2 452 392
- US-A- 4 911 159
- US-A- 5 318 565
- US-A1- 2005 124 986
- US-A1- 2012 203 165

## Description

### BACKGROUND

### 1. Technical Field

The present disclosure relates generally to electrosurgical instruments and, more particularly, to an improved electrosurgical dissector having a telescoping aspiration cannula.

### 2. Background of Related Art

Electrosurgical instruments which dissect tissue and have provisions for evacuating fluid and/or smoke have been available for some time. Typically, a combination electrosurgery and suction device is employed wherever excessive fluid or smoke must be removed from the operative site in order to successfully perform the desired procedure. Generally, these devices include a housing or handle having an electrode extending from a distal end thereof, and a suction port disposed in proximity to the electrode to evacuate fluid and/or smoke. A suction source is attached to the instrument for evacuating excess fluid, debris, smoke, vapors, etc. from the surgical site through the suction port. The electrode is operably coupled to a source of electrosurgical energy, such as an electrosurgical generator.

Existing electrosurgical dissectors may have drawbacks. For example, the configuration of an instrument's electrode and suction port may not be well-suited for all phases of a procedure, necessitating time-consuming instrument changes during surgery.

WO2014/032157 discloses an adjustable electrosurgical pencil with a slidably adjustable vacuum tube and a slidably adjustable electrode mounted therein.

### SUMMARY

The invention is defined in appended independent claim 1, preferred embodiments are described in the dependent claims. In one aspect, the present disclosure is directed to an electrosurgical dissection instrument. In an exemplary embodiment, the disclosed electrosurgical dissection instrument includes a housing defining a longitudinal axis, at least one vacuum cannula extending distally from the housing and selectively movable relative to the housing along the longitudinal axis, and an electrode extending distally from the housing through the at least one vacuum cannula and selectively movable relative to the housing along the longitudinal axis. In some embodiments, disclosed electrosurgical dissection instrument includes an aspiration tube having a proximal end and a distal end that is configured to operatively couple to the at least one vacuum cannula.

In embodiments, the aspiration tube is permanently coupled, integrally formed or selectively coupled to (e.g. removable with respect to) the vacuum tube. The vacuum cannula and the aspiration tube may include removable coupling means to allow them to be removably coupled to one another. A proximal end of the aspiration tube may be removably couple to the distal end of the vacuum cannula. In embodiments, the aspiration tube extends distally beyond the vacuum cannula.

In embodiments, the aspiration tube includes an open distal end providing an aspiration port.

In embodiments, the aspiration tube has a beveled distal end.

In embodiments, the aspiration tube and the vacuum cannula move in conjunction.

In embodiments, the instrument has a configuration in which the electrode is extended beyond a distal end of the vacuum cannula. In embodiments including the aspiration tube, the instrument has a configuration in which the electrode is extended beyond a distal end of the aspiration tube.

In embodiments, the instrument has a configuration in which electrode distal end is retracted relative to a distal end of the aspiration tube.

In embodiments, the instrument has a configuration in which a distal end of the electrode is extended a first distance beyond a distal end of the aspiration tube and a configuration in which the electrode is extended a second greater distance beyond a distal end of the aspiration tube.

In some embodiments, the aspiration tube defines at least one pre-aspiration port formed in proximity to a distal end of the aspiration tube. In some embodiments, aspiration tube is transparent. In some embodiments, a surface (e.g. an inner surface) of the aspiration tube includes a hydrophobic coating, an oleophobic coating, and/or a lubricious coating.

In some embodiments, the electrosurgical dissection instrument includes an aspiration tip disposed at a distal end of the aspiration tube. The aspiration tip may be formed from elastomeric material and/or a rigid material. That is, the aspiration tip may be formed of a relatively elastomeric material as compared to the aspiration tube. The material may be silicone.

In some embodiments, the at least one vacuum cannula is configured to operatively couple to a vacuum source, and the electrode is configured to operatively couple to a source of electrosurgical energy. In some embodiments, the electrosurgical dissection instrument includes a vacuum actuator configured to activate a vacuum source. In some embodiments, the electrosurgical dissection instrument includes an electrosurgical actuator configured to activate a source of electrosurgical energy. According to the invention, the housing of the electrosurgical dissection instrument includes a cannula slide control operatively coupled to the at least one vacuum cannula, and which is configured to move the at least one vacuum cannula along a longitudinal axis of the instrument.

According to the invention, the housing includes an electrode slide control operatively coupled to the electrode and configured to move the electrode along a longitudinal axis of the instrument. In some embodiments, the at least one vacuum cannula includes a first vacuum cannula and a second vacuum cannula coaxially disposed within the first vacuum cannula. The first and second cannulas extend distally from the housing and are selectively movable relative to each other and the housing along the longitudinal axis.

According to the invention, the vacuum cannula and the electrode are independently and selectively positionable with respect to the housing and, therefore, with respect to each other.

In another aspect of the present disclosure, embodiments of an electrosurgical dissection system are described. In an exemplary embodiment, the disclosed electrosurgical dissection system includes an electrosurgical generator, a vacuum source, and an electrosurgical dissection instrument configured for operable engagement with the electrosurgical generator and the vacuum source. The electrosurgical dissection instrument includes a housing defining a longitudinal axis and at least one vacuum cannula. The at least one vacuum cannula extends distally from the housing, and is configured for selective positioning along the longitudinal axis. The electrosurgical dissection instrument further includes an electrode that extends distally from the housing through the at least one vacuum cannula, and is configured for selective positioning along the longitudinal axis.

In some embodiments, the at least one vacuum cannula is configured to operatively couple to the vacuum source, and the electrode is configured to operatively couple to the electrosurgical generator.

In some embodiments, the electrosurgical dissection instrument of the electrosurgical dissection system includes a vacuum actuator disposed on an outer surface of the housing that is configured to activate the vacuum source. In some embodiments, the electrosurgical dissection instrument of the electrosurgical dissection system includes an electrosurgical actuator disposed on an outer surface of the housing that is configured to activate the electrosurgical generator.

In some embodiments, the electrosurgical dissection instrument of the electrosurgical dissection system includes a cannula slide control that is operatively coupled to the at least one vacuum cannula, and is configured to move the at least one vacuum cannula along the longitudinal axis. In some embodiments, the electrosurgical dissection instrument of the electrosurgical dissection system includes an electrode slide control operatively coupled to the electrode that is configured to move the electrode along the longitudinal axis.

In some embodiments, the at least one vacuum cannula includes a first vacuum cannula, and a second vacuum cannula coaxially disposed within the first vacuum cannula. The first and second cannulas extend distally from the housing and are selectively movable relative to each other and the housing along the longitudinal axis.
In yet another aspect of the present disclosure, an example of an electrosurgical dissection instrument includes a housing defining a longitudinal axis, and first and second vacuum cannulas configured to operatively couple to a vacuum source and which extend distally from the housing. The second vacuum cannula is coaxially disposed within the first vacuum cannula. The first and second vacuum cannulas are selectively movable relative to each other, and to the housing, along the longitudinal axis. The electrosurgical dissection instrument includes an electrode configured to operatively couple to a source of electrosurgical energy which extends distally from the housing through the vacuum cannula, and is selectively movable relative to the housing along the longitudinal axis. The electrosurgical dissection instrument includes a vacuum actuator configured to activate the vacuum source and an electrosurgical actuator configured to activate a source of electrosurgical energy. The electrosurgical dissection instrument includes a cannula position control operatively coupled to the first and second vacuum cannulas that is configured to move at least one of the first vacuum cannula or the second vacuum cannula along the longitudinal axis. The electrosurgical dissection instrument includes an electrode slide operatively coupled to the electrode and configured to move the electrode along the longitudinal axis.

### BRIEF DESCRIPTION OF THE DRAWINGS

The above and other aspects, features, and advantages of the present disclosure will become more apparent in light of the following detailed description when taken in conjunction with the accompanying drawings in which:
Fig. 1 is a perspective view of an illustrative embodiment of an electrosurgical dissection system in accordance with the present disclosure;
Fig. 2 is a side cutaway view of a further illustrative embodiment of an electrosurgical dissection instrument in accordance with the present disclosure;
Fig. 3 is a perspective partially exploded view of yet another illustrative not claimed example of an electrosurgical dissection instrument in accordance with the present disclosure;
Fig. 4 is a perspective view of the electrosurgical dissection instrument example of Fig. 3 showing an electrode in an extended position;
Fig. 5A is a perspective view of the electrosurgical dissection instrument of Fig. 3 showing an electrode and an aspiration tube in a retracted position;
Fig. 5B is a perspective view of the electrosurgical dissection instrument of Fig. 3 showing an electrode in an extended position an aspiration tube in a retracted position;
Fig. 6 is a view of a distal end of an embodiment of an aspiration tube having an aspiration tip in accordance with the present disclosure;
Fig. 7A is a cross-sectional view of a distal end of another embodiment of an aspiration tube joined to an aspiration tip using an overmolded lap joint configuration in accordance with the present disclosure;
Fig. 7B is a cross-sectional view of a distal end of an embodiment of an aspiration tube joined to an aspiration tip using a slip joint configuration in accordance with the present disclosure;
Fig. 7C is an enlarged, cross-sectional view of a distal end of an embodiment of an aspiration tube removably joined to an aspiration tip using a snap fit configuration in accordance with the present disclosure;
Fig. 8A is a side cutaway view of another illustrative embodiment of an electrosurgical dissection instrument in accordance with the present disclosure having a first movable aspiration tube and a second movable aspiration tube;
Fig. 8B is a side cutaway view of the Fig. 8A embodiment of an electrosurgical dissection instrument showing the first movable aspiration tube in a retracted position and the second movable aspiration tube in an extended position; and
Fig. 8C is a side cutaway view of the Fig. 8A embodiment of an electrosurgical dissection instrument showing the first movable aspiration tube in an extended position and the second movable aspiration tube in a retracted position.

### DETAILED DESCRIPTION

Particular illustrative embodiments of the present disclosure are described hereinbelow with reference to the accompanying drawings; however, the disclosed embodiments are merely examples of the disclosure, which may be embodied in various forms. Well-known functions or constructions and repetitive matter are not described in detail to avoid obscuring the present disclosure in unnecessary or redundant detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

As shown in the drawings and as described throughout the following description, and as is traditional when referring to relative positioning on an object, the term "proximal" refers to the end of the apparatus that is closer to the user and the term "distal" refers to the end of the apparatus that is further from the user. In addition, as used herein in the description and in the claims, terms referencing orientation, e.g., "top", "bottom", "upper", "lower", "left", "right", and the like, are used with reference to the figures and features shown and described herein. It is to be understood that embodiments in accordance with the present disclosure may be practiced in any orientation without limitation. In this description, as well as in the drawings, like-referenced numbers represent elements which may perform the same, similar, or equivalent functions. The word "exemplary" is used herein to mean "serving as an example, instance, or illustration." Any embodiment described herein as "exemplary" is not necessarily to be construed as preferred or advantageous over other embodiments. The word "example" may be used interchangeably with the term "exemplary."

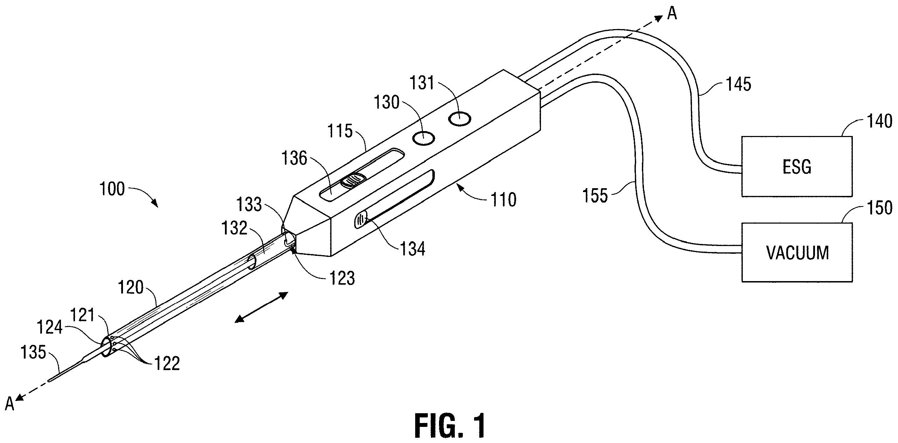

With reference to Fig. 1, an electrosurgical dissection system 100 is presented having an electrosurgical dissection instrument 110 that is operably coupled to an electrosurgical generator 140, labeled as "ESG" in Fig.1 via a conductor 145. Electrosurgical dissection instrument 110 is operably coupled to a vacuum source 150 by a lumen 155. Dissection instrument 110 includes a housing 115 disposed at the proximal end thereof, and an aspiration tube 120 extending distally from the housing 115 and having an electrode 135 extending distally therethrough. Conductor 145 and lumen 155 are sealed at the respective entry points thereof into housing 115 to prevent fluid intrusion from the surgical site into the housing 115 and/or to prevent vacuum leaks. As described in detail below, aspiration tube 120 and/or electrode 135 are independently and selectively positionable with respect to housing 115 and, therefore, with respect to each other. Advantageously, this enables dissection instrument 110 to be dynamically reconfigured during use to accommodate a wide variety of interventional scenarios.

In embodiments, aspiration tube 120 may be formed from material having optically-transparent properties, for example, and without limitation, polycarbonate, to allow a surgeon to visually monitor the aspiration process. This, in turn, enables the surgeon to detect blockages, quantitatively and qualitatively assess aspirated material, and to improve the effectiveness of the procedure and of dissection instrument 110. Aspiration tube 120 includes an inner surface 124 that is polished and/or includes a transparent coating, such as a hydrophobic and/or oleophobic coating that is adapted to repel aspirated materials to promote visual clarity and/or improve the flow of aspirated materials. In embodiments, aspiration tube 120 may include a lubricious coating, such as, without limitation, polytetrafluoroethylene (PTFE). One such PTFE coating is sold under the brand name Teflon® and is available from E. I. du Pont de Nemours and Company or its affiliates.

In embodiments, the lubricious coating may be transparent and/or applied in a manner so as to render the lubricious coating effectively transparent, such as, without limitation, nano-coating.

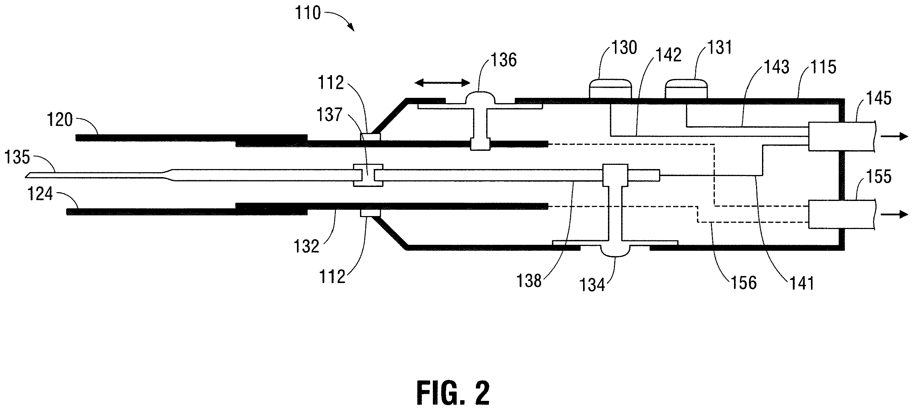

Referring to Figs. 1 and 2, housing 115 includes a number of user interface elements which enable a surgeon to manipulate and/or control dissection instrument 110. Housing 115 includes an electrosurgical actuator 130 disposed on an outer surface thereof that is operatively coupleable to electrosurgical generator 140 by suitable known techniques to selectively activate the delivery of electrosurgical energy to electrode 135. Housing 115 also includes a vacuum actuator 131 disposed on an outer surface of housing 115 that is operatively coupleable to vacuum source 150 by suitable known techniques to selectively activate the application of aspiration suction to dissection instrument 110 and/or to aspiration tube 120. Electrosurgical actuator 130 and/or vacuum actuator 131 may include a handswitch, such as a pushbutton (e.g., for on/off operation) as is shown in Fig. 1, and/or may include a variable control, such as a pressure sensor, rotary control, or slide control (not shown), that configured to continuously vary a property of the delivered electrosurgical energy and/or the amount of suction.

An electrosurgical control conductor 142 and a vacuum control conductor 143 provide electrical communication between electrosurgical actuator 130 and vacuum actuator 131, respectively, to the electrosurgical generator 140 and vacuum source 150, respectively. Electrosurgical actuator 130 and vacuum actuator 131 are sealed relative the housing 115 to prevent fluid intrusion from the surgical site into the housing 115 and/or to prevent vacuum leaks.

With continuing reference to Fig. 2, dissection instrument 110 includes a selectively positionable vacuum cannula 132 extending from a distal end of housing 115. Vacuum cannula 132 is configured for selective engagement with aspiration tube 120 to enable aspiration tube 120 to be easily attachable, detachable, and/or replaceable as desired. Vacuum cannula 132 includes one or more bayonet-style mounting lugs 133 extending radially from a surface thereof that are configured to operatively engage one or more mating bayonet-style mounting slots 123 defined in a proximal end of aspiration tube 120. In some embodiments, aspiration tube 120 may couple to vacuum cannula 132 using additional or alternative suitable techniques, including without limitation, a threaded coupling, an interference or friction coupling, a mechanical latch, a snap coupling, a magnetic coupling, electromechanical coupling, and so forth. In some embodiments, aspiration tube 120 and vacuum cannula 132 may be permanently coupled and/or integrally formed.

Aspiration tube 120 includes a beveled distal end 121. One or more pre-aspiration vents 122 (Fig. 1) are defined in proximity to distal end 121 of aspiration tube 120. The one or more pre-aspiration vents 122 provide an alternative suction inlet into aspiration tube 120 to prevent the blockage of beveled distal end 121 by, e.g., a suddenly-captured portion of tissue. Advantageously, the combination of beveled distal end 121 and the one or more pre-aspiration vents 122 have been found to reduce or eliminate undesirable tissue trauma which may result from tissue being inadvertently being caught and/or sucked into aspiration tube 120. In addition, the beveled distal end 121 of aspiration tube 120 provides more working space around electrode 135, and may improve visibility at the operative site, further enhancing utility and ease-of-use. Beveled distal end 121 may be configured to accept an aspiration tip, which may be formed from elastomeric and/or rigid material, as described hereinbelow.

Vacuum cannula 132 is configured for selective positioning along a longitudinal axis (A-A) of dissection instrument 110. As shown in the embodiment of Figs. 1 and 2, vacuum cannula 132 is coupled to a cannula slide control 136 that is configured to enable a surgeon to slide vacuum cannula 132 in a distal and/or proximal direction as desired. Cannula slide control 136 may include a locking, friction-fit, detent, and/or other suitable arrangement which retains vacuum cannula 132 in a desired position. In some embodiments, vacuum cannula 132 may utilize a twist-lock mechanism where rotating vacuum cannula 132 to a first position that loosens vacuum cannula 132 from housing 115 thereby allowing vacuum cannula 132 to be adjusted longitudinally as desired, and where rotating vacuum cannula 132 to a second position secures vacuum cannula 132 in a desired position. A gasket 112 provides a vacuum seal and fluid seal between vacuum cannula 132 and housing 115 while permitting vacuum cannula 132 to slide longitudinally with respect to housing 115. In this manner, gasket 112 helps ensure that vacuum is applied to the surgical site only through vacuum cannula 132 and/or aspiration tube 120. In some embodiments, cannula slide control 136 may be configured to enable a surgeon to slide, independently or in tandem, a plurality of cannulas in a distal and/or proximal direction as desired. In yet other embodiments, an instrument in accordance with the present disclosure may include a plurality of cannula slide controls 136, each configured to enable a surgeon to slide a respective cannula in a distal and/or proximal direction as desired.

During use, aspiration tube 120 may be joined to vacuum cannula 132 by sliding aspiration tube 120 onto vacuum cannula 132 and engaging the one or more bayonet lugs 133 with the more mating bayonet slots 123, or using other alternative joining techniques as described above. Thus, the combination of aspiration tube 120 and vacuum cannula 132 may be adjusted independently or in combination, as required, to enable a surgeon to configure the aspiration aspects of dissection instrument 110. Cannula slide control 136 and electrode slide control 134 are sealed to prevent fluid intrusion from the surgical site into the housing 115 and/or to prevent vacuum leaks.

With continued reference to Figs. 1 and 2, electrode 135 extends distally from housing 115, and is generally centered within aspiration tube 120 and/or vacuum cannula 132. In the illustrated embodiment, electrode 135 is configured for selective positioning along a longitudinal axis of dissection instrument 110. In the presently-described embodiment, electrode 135 is configured for selective electromechanical attachment and/or detachment to an electrode support 138 disposed within housing 155 via an electrode coupler 137. Electrode support 138, in turn, is operably coupled to electrode slide control 134. In embodiments, at least a portion of electrode support 138 and electrode coupler 137 are formed from electrically-conductive materials. Electrode slide control 134 is configured to enable a surgeon to move electrode 135 in a distal and/or proximal direction as desired. Slide control 134 may include a locking, friction-fit, detent, and/or other suitable arrangement to retain electrode support 138 and electrode 135 in a desired position. Electrode 135 may engage electrode coupler 137 via a threaded attachment, a bayonet attachment, a friction-fit/interference attachment, or any other suitable arrangement. An electrosurgical delivery conductor 141 provides electrical communication between electrode support 138 and electrosurgical generator 140.

In some embodiments, electrode 135 may be configured to extend distally from housing 115 by a fixed amount, e.g., in a "fixed length" configuration, which may be preferable in view of particular surgical requirements, and/or may offer cost savings in applications where a variable electrode length is not required.

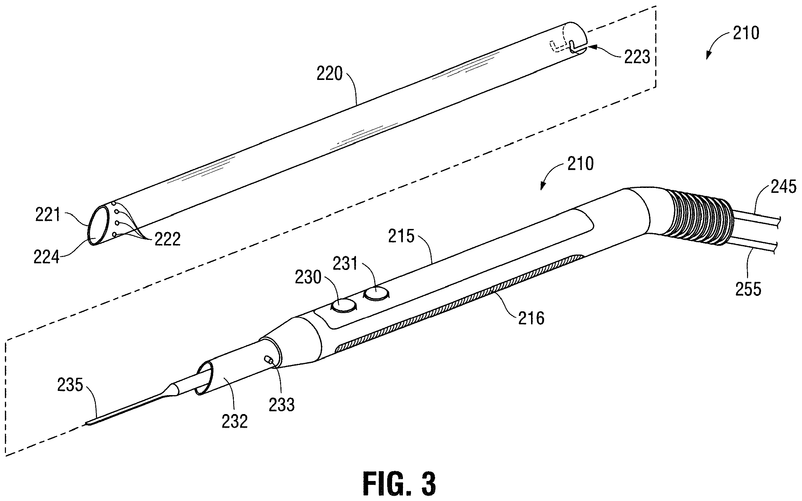

Turning now to Figs. 3, 4, 5A, and 5B, further exemplary examples of an electrosurgical dissection instrument 210 in various configurations are illustrated. Electrosurgical dissection instrument 210 includes a housing 215 having a selectively positionable vacuum cannula 232 extending from a distal end thereof. A selectively positionable electrode 235 extends through vacuum cannula 232 from housing 215. Housing 215 includes one or more ergonomic features 216 which may include, without limitation, serrations, knurling, and/or overmolded elastomeric grip-enhancing material. Electrosurgical dissection instrument 210 is configured to operably couple to an electrosurgical generator via a conductor 245, and to a vacuum source by a lumen 255. Conductor 245 and/or lumen 255 may include one or more control conductors and/or energy delivery conductors as described above. Housing 215 includes electrosurgical actuator 230 and vacuum actuator 231 that are configured to activate a source of electrosurgical energy and vacuum suction, respectively. Electrosurgical dissection instrument 210 includes a selectively-attachable aspiration tube 220. Aspiration tube 220 includes one or more bayonet slots 223 at a proximal end thereof that are configured to engage mating one or more bayonet lugs 233 disposed on vacuum cannula 232. Aspiration tube includes a beveled distal end 221 having one or more pre-aspiration ports 222 defined therein, and an inner surface 224 that is polished and/or includes a transparent coating, such as a hydrophobic and/or oleophobic coating that is adapted to repel aspirated materials to promote visual clarity and/or the flow of aspirated material.

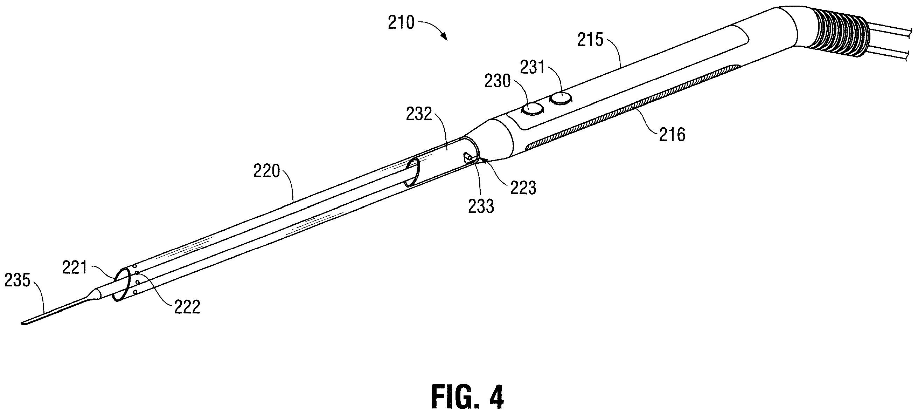

As seen in Fig. 3, electrosurgical dissection instrument 210 may be configured with aspiration tube 220 removed therefrom. In the configuration shown in Fig. 3, electrode 235 is extended distally beyond a distal end 225 of vacuum cannula 232. This configuration may be advantageous, where, for example, a procedure does not require an extended reach and/or calls for very close and controlled manipulation of electrode 235 with respect to tissue. A more extended configuration of electrosurgical dissection instrument 210 is shown in Fig. 4, wherein aspiration tube 220 is attached to vacuum cannula 232. Here, electrode 235 is extended distally beyond beveled distal end 221 of aspiration tube 220. This configuration may be advantageous where an extended reach is required, for example, into an abdominal pneumoperitoneum. In this configuration, the more proximal position of distal end 221 of aspiration tube 220 with respect to electrode 235 may be desirable to evacuate smoke generated during an electrosurgical procedure.

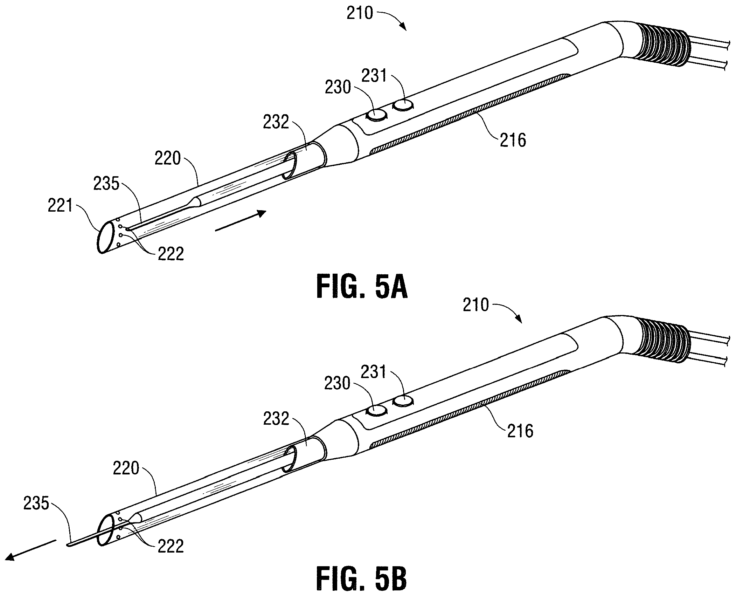

Turning now to Figs. 5A and 5B, two additional alternative configurations of electrosurgical dissection instrument 210 are illustrated. As shown in Fig. 5A, electrode 235 has been retracted to a position proximal of beveled distal end 221 of aspiration tube 220. This configuration may be desirable where, for example, fluid, tissue, and/or other surgical debris need to be aspirated from the surgical site. In Fig. 5B, electrode 235 is extended slightly distal of beveled distal end 221 of aspiration tube 220. This configuration may be desirable where, for example, targeted tissue needs to be treated electrosurgically while concurrently, smoke, fluid, tissue, and/or other surgical debris needs to be aspirated from the surgical site. This configuration may be beneficial during, for example, resection procedures where continuous dissection, cauterization, and aspiration is required.

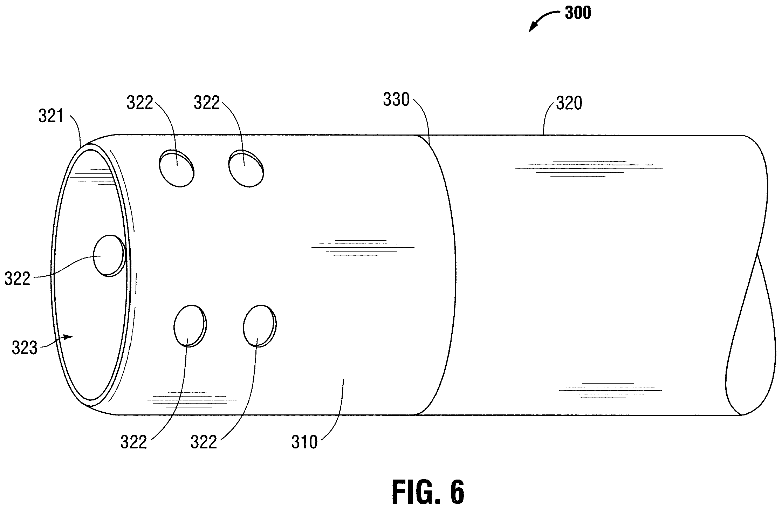

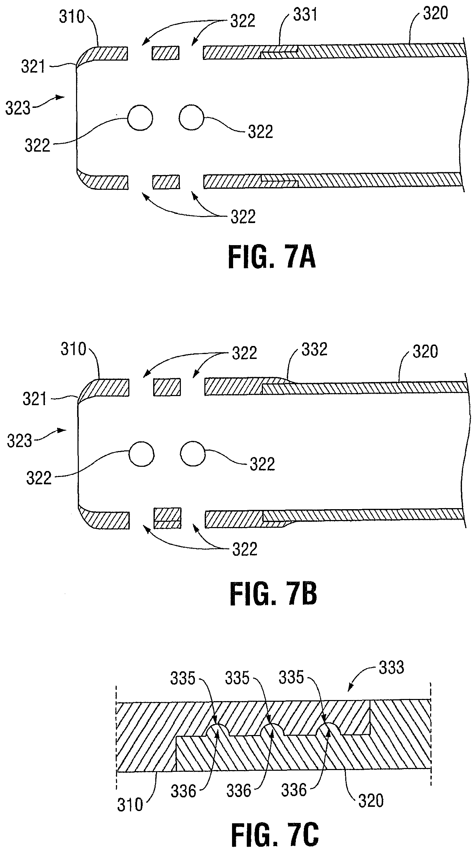

With attention now to Figs. 6 and 7A-C, another embodiment of an aspiration tube assembly 300 in accordance with the present disclosure includes an aspiration tip 310 that is joined to a distal end of a rigid or semi-rigid aspiration tube 320. In the present example embodiment, aspiration tip 310 is formed from an elastomeric material capable of withstanding the elevated temperatures associated with electrosurgical dissection, including without limitation high-temperature silicone. Advantageously, the use of elastomerics such as silicone may reduce the likelihood of biomaterials from adhering to tip 310, which facilitates the effective execution of dissection procedures and may reduce or prevent the occurrence of clogging. In addition, the use of elastomeric material may reduce trauma to surrounding anatomical structures, and may facilitate surgical techniques which are difficult or impossible to execute with rigid aspiration tip structures. In other embodiments, aspiration tip 310 is formed from a rigid material which may be suitable for use in procedures involving, for example, blunt dissection.

Aspiration tip 310 includes a distal end 321 having a rounded profile that may further ensure that the use of tube assembly 300 is atraumatic with respect to tissue adjacent to the operative site. In some embodiments, distal end 321 may include a beveled, tapered, or blunt profile. Aspiration tip 310 includes a primary aspiration port 323, and one or more pre-aspiration vents 322 defined in the wall of resilient tip 310. Pre-aspiration vents 322 provide an alternative suction inlet into aspiration tube assembly 300 in the event a portion of tissue or other biomaterial blocks aspiration port 323.

Aspiration tip 310 is joined to aspiration tube 320 at a junction 330. In some embodiments, it is envisioned that aspiration tip 310 is permanently joined to aspiration tube 320, while in other embodiments, aspiration tip 310 may be selectively joined (e.g., interchangeable or replaceable) with aspiration tube 320. In the example embodiment shown in Fig. 7A, aspiration tip 310 is formed onto aspiration tube 320 by overmolding using a lap joint 331 configuration. In this embodiment, the outer diameters of aspiration tip 310 and aspiration tube 320 are substantially the same, with no gaps or discontinuities at junction 330.

In the example embodiment shown in Fig. 7B, aspiration tip 310 is formed onto aspiration tube 320 by overmolding using a slip joint 332 configuration. This embodiment may be more cost-effective to manufacture, since no special machining or forming is required at a distal end of aspiration tube 320, e.g., simple flat-cut tube stock may be employed to produce aspiration tube 320. In the example embodiment shown in Fig. 7C, aspiration tip 310 is selectively and/or removably joinable with aspiration tube 320. In this embodiment, a lap joint configuration 333 is employed in which one or more annular ridges 336 provided by aspiration tube 320 engage a corresponding one or more annular grooves 335 defined in aspiration tip 310 to secure aspiration tip 310 to aspiration tube 320 via a snap fit. In other embodiments, aspiration tip 310 may be joined to aspiration tube 320 by threaded attachment, press fit, twist or bayonet-style coupling, and/or any other suitable manner of attachment.

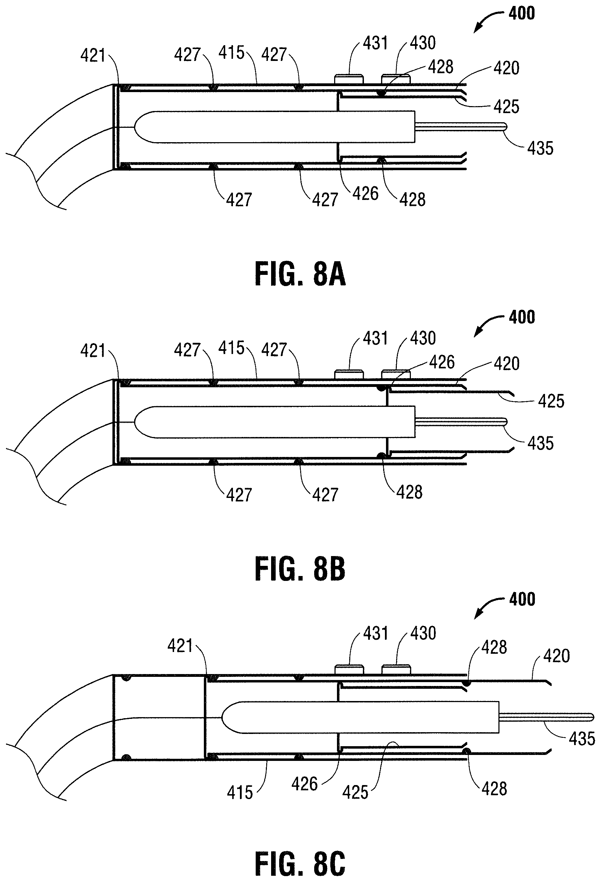

Referring now to Figs. 8A-C, an example embodiment of a dissection instrument 400 in accordance with the present disclosure includes a housing 415, a first cannula 420 that is selectively extendable from a distal end of the housing 415, a second cannula 425 coaxially disposed within the first cannula 420 and selectively extendable from a distal end of the housing 415, and an electrode 435 coaxially disposed within the first and second cannulas 420, 425 and selectively extendable from a distal end of the housing 415.

Housing 415 includes an electrosurgical actuator 430 disposed on an outer surface thereof that is operatively coupleable to a source of electrosurgical energy by any suitable techniques to selectively activate the delivery of electrosurgical energy to electrode 435. Housing 415 also includes a vacuum actuator 431 disposed on an outer surface of housing 415 that is operatively coupleable to a vacuum source by any suitable technique to selectively activate the application of aspiration suction to dissection instrument 400, e.g., to aspiration tubes 420 and/or 425. Electrosurgical actuator 430 and/or vacuum actuator 431 may include a handswitch, such as a pushbutton (e.g., for on/off operation) as is shown in Fig. 1, and/or may include a variable control, such as a pressure sensor, rotary control, or slide control (not shown, e.g., to continuously vary a property of the delivered electrosurgical energy and/or the amount of suction).

In the example embodiment illustrated in Figs. 8A-C, a first cannula 420 has an overall length that is greater than the overall length of second cannula 425. In other embodiments, first cannula 420 and second cannula 425 may have similar lengths or second cannula 425 may have an overall length that is greater than the overall length of first cannula 420.

First cannula 420 includes an annular rib 421 disposed at a proximal end thereof that is configured to engage an inner surface of housing 415. In embodiments, annular rib 421 forms a vacuum seal to reduce or eliminate any vacuum leakage from first cannula 420. Additionally or alternatively, annular rib 421 is configured to engage one or more detents 427 provided on an inner surface of housing 415, which enables a user to securely retain first cannula 420 at a desired position. The one or more detents 427 may be arranged to provide one or more preset positions at which to secure first cannula 420. Additionally or alternatively, the one or more detents 427 may be arranged in a closely-spaced or ratchet-like configuration to enable substantially continuous adjustment of first cannula 420. In embodiments, annular rib 421 and/or the one or more detents 427 are configured to provide an audible click and/or tactile feedback to the user as first cannula 420 is moved into engagement at each detent position.

Second cannula 425 includes an annular rib 426 disposed at a proximal end thereof that is configured to engage an inner surface of first cannula 420. In embodiments, annular rib 426 forms a vacuum seal to reduce or eliminate any vacuum leakage from second cannula 425. Additionally or alternatively, annular rib 426 is configured to engage one or more detents 428 provided on an inner surface of first cannula 420, which enables a user to securely retain second cannula 425 at a desired position. The one or more detents 428 may be arranged to provide one or more preset positions at which to secure second cannula 425. Additionally or alternatively, the one or more detents 428 may be arranged in a closely-spaced or ratchet-like configuration to enable substantially continuous adjustment of second cannula 425. In embodiments, annular rib 426 and/or the one or more detents 428 are configured to provide an audible click and/or tactile feedback to the user as second cannula 425 is moved into engagement at each detent position.

In embodiments, as seen in Fig. 8A, electrode 435 is in a partially-extended position whereby a distal end of electrode 435 extends distal from a distal end of housing 415. In addition, first cannula 420 and second cannula 425 are in a fully-retracted position whereby a distal end of first cannula 420 and a distal end of second cannula 425 are substantially aligned with a distal end of housing 415.

In Fig. 8B, electrode 435 is in the partially-extended position of Fig. 8A and second cannula 425 is in a fully-extended position whereby a distal end of second cannula 425 extends distally beyond a distal end of housing 415 and is substantially aligned with a distal end of electrode 435. First cannula 420 remains in the fully-retracted position of Fig 8A.

In Fig. 8C, electrode 435 is in a fully-extended position of Fig. 8A and first cannula 420 is in an extended position whereby a distal end of first cannula 420 extends distally beyond a distal end of housing 415. In this example illustration, a distal end of electrode 435 extends distally beyond a distal end of first cannula 420. Second cannula 425 remains in a fully-retracted position.

Other configurations of dissection instrument 400 are contemplated within the scope of the present disclosure, e.g., various alternative positions of first cannula 420, second cannula 425, and electrode 435. In addition, electrode 435 may be replaceable to enable electrodes of differing lengths and profiles to be utilized, and first cannula 420 and/or second cannula 425 may be replaceable to enable use of aspiration tubes of differing lengths and/or diameter, having one or more pre-aspiration ports, having an elastomeric or rigid aspiration tip, and so forth.

The described embodiments of the present disclosure are intended to be illustrative rather than restrictive, and are not intended to represent every embodiment of the present disclosure.

## Claims

1. An electrosurgical dissection instrument (110), comprising:
a housing (115) defining a longitudinal axis;
at least one vacuum cannula (132) extending distally from the housing and selectively movable relative to the housing along the longitudinal axis; and
an electrode (135) extending distally from the housing through the at least one vacuum cannula and selectively movable relative to the housing along the longitudinal axis,
**characterized in that** the housing comprises:
a cannula slide control (136) operatively coupled to the at least one vacuum cannula and configured to move the at least one vacuum cannula along the longitudinal axis; and
an electrode slide control (134) operatively coupled to the electrode and configured to move the electrode along the longitudinal axis,
whereby the vacuum cannula and the electrode are independently and selectively positionable with respect to the housing and each other by using the cannula slide control (136) and the electrode slide control (134).

2. The electrosurgical dissection instrument in accordance with claim 1, further comprising an aspiration tube (120) having a proximal end and a distal end (121), configured to operatively couple to the at least one vacuum cannula (132).

3. The electrosurgical dissection instrument in accordance with claim 2, wherein the aspiration tube (120) defines at least one pre-aspiration port (122) formed in proximity to the distal end thereof.

4. The electrosurgical dissection instrument in accordance with claim 2 or 3, wherein the aspiration tube (120) is transparent.

5. The electrosurgical dissection instrument in accordance with claim 2, 3 or 4 wherein a surface of the aspiration tube (120) includes a coating selected from the group consisting of a hydrophobic coating, an oleophobic coating, and a lubricious coating.

6. The electro surgical dissection instrument in accordance with claim 2, 3, 4 or 5 further comprising an aspiration tip (310) disposed at a distal end of the aspiration tube (320), wherein the aspiration tip is formed from a material selected from the group consisting of an elastomeric material and a rigid material.

7. An electrosurgical dissection instrument according to any preceding claim, further comprising:
an electrosurgical generator (140); and
a vacuum source (150),
wherein the the at least one vacuum cannula (132) is operatively coupled to the vacuum source and the electrode (135) is operatively coupled to the electrosurgical generator.

8. The electrosurgical dissection instrument in accordance with claim 7, further comprising a vacuum actuator (131) disposed on an outer surface of the housing and configured to activate the vacuum source.

9. The electrosurgical dissection instrument in accordance with claim 7 or8, further comprising an electrosurgical actuator (130) disposed on an outer surface of the housing and configured to activate the electrosurgical generator.

10. The electrosurgical dissection instrument in accordance with claim 1, wherein the at least one vacuum cannula includes a first vacuum cannula (420) and a second vacuum cannula (425) coaxially disposed within the first vacuum cannula and the first and second cannulas extend distally from the housing (415) and are selectively movable relative to each other and the housing (415) along the longitudinal axis.

## Patentansprüche

1. Elektrochirurgisches Dissektionsinstrument (110), umfassend:
ein Gehäuse (115), das eine Längsachse definiert;
mindestens eine Vakuumkanüle (132), die sich distal von dem Gehäuse erstreckt und selektiv relativ zum Gehäuse entlang der Längsachse beweglich ist; und
eine Elektrode (135), die sich distal von dem Gehäuse durch die mindestens eine Vakuumkanüle erstreckt und selektiv relativ zum Gehäuse entlang der Längsachse beweglich ist,
**dadurch gekennzeichnet, dass** das Gehäuse umfasst:
eine Kanülenschiebesteuerung (136), die betriebsmäßig mit der mindestens einen Vakuumkanüle gekoppelt und konfiguriert ist, um die mindestens eine Vakuumkanüle entlang der Längsachse zu bewegen; und
eine Elektrodenschiebesteuerung (134), die betriebsmäßig mit der Elektrode gekoppelt und konfiguriert ist, um die Elektrode entlang der Längsachse zu bewegen,
wobei die Vakuumkanüle und die Elektrode unter Verwendung der Kanülenschiebesteuerung (136) und der Elektrodenschiebesteuerung (134) unabhängig und selektiv in Bezug auf das Gehäuse und zueinander positionierbar sind.

2. Elektrochirurgisches Dissektionsinstrument nach Anspruch 1, weiter umfassend ein Aspirationsrohr (120) mit einem proximalen Ende und einem distalen Ende (121), das konfiguriert ist, um sich betriebsmäßig mit der mindestens einen Vakuumkanüle (132) zu koppeln.

3. Elektrochirurgisches Dissektionsinstrument nach Anspruch 2, wobei das Aspirationsrohr (120) mindestens eine Voraspirationsöffnung (122), die in der Nähe des distalen Endes davon ausgebildet ist, definiert.

4. Elektrochirurgisches Dissektionsinstrument nach Anspruch 2 oder 3, wobei das Aspirationsrohr (120) transparent ist.

5. Elektrochirurgisches Dissektionsinstrument nach Anspruch 2, 3 oder 4, wobei eine Oberfläche des Aspirationsrohr (120) eine Beschichtung aufweist, die aus der Gruppe ausgewählt ist, die aus einer hydrophoben Beschichtung, einer oleophoben Beschichtung und einer schmierenden Beschichtung besteht.

6. Elektrochirurgisches Dissektionsinstrument nach Anspruch 2, 3, 4 oder 5, weiter umfassend eine Aspirationsspitze (310), die an einem distalen Ende des Aspirationsrohrs (320) angeordnet ist, wobei die Aspirationsspitze aus einem Material gebildet ist, das aus der Gruppe ausgewählt ist, die aus einem elastomeren Material und einem starren Material besteht.

7. Elektrochirurgisches Dissektionsinstrument nach einem vorstehenden Anspruch, weiter umfassend:
einen elektrochirurgischen Generator (140); und
eine Vakuumquelle (150),
wobei die mindestens eine Vakuumkanüle (132) betriebsmäßig mit der Vakuumquelle gekoppelt ist und die Elektrode (135) betriebsmäßig mit dem elektrochirurgischen Generator gekoppelt ist.

8. Elektrochirurgisches Dissektionsinstrument nach Anspruch 7, weiter umfassend ein Vakuumstellglied (131), das an einer Außenfläche des Gehäuses angeordnet und konfiguriert ist, um die Vakuumquelle zu aktivieren.

9. Elektrochirurgisches Dissektionsinstrument nach Anspruch 7 oder 8, weiter umfassend ein elektrochirurgisches Stellglied (130), das an einer Außenfläche des Gehäuses angeordnet und konfiguriert ist, um den elektrochirurgischen Generator zu aktivieren.

10. Elektrochirurgisches Dissektionsinstrument nach Anspruch 1, wobei die mindestens eine Vakuumkanüle eine erste Vakuumkanüle (420) und eine zweite Vakuumkanüle (425) aufweist, die koaxial innerhalb der ersten Vakuumkanüle angeordnet ist, und sich die ersten und zweiten Kanülen distal von dem Gehäuse (415) erstrecken und selektiv relativ zueinander und in Bezug auf das Gehäuse (415) entlang der Längsachse beweglich sind.

## Revendications

1. Instrument de dissection électrochirurgical (110), comprenant :
un boîtier (115) définissant un axe longitudinal ;
au moins une canule à vide (132) s'étendant de manière distale depuis le boîtier et mobile sélectivement par rapport au boîtier le long de l'axe longitudinal ; et
une électrode (135) s'étendant de manière distale depuis le boîtier à travers la au moins une canule à vide et mobile sélectivement par rapport au boîtier le long de l'axe longitudinal,
**caractérisé en ce que** le boîtier comprend :
une commande coulissante de canule (136) couplée en service à la au moins une canule à vide et configurée pour déplacer la au moins une canule à vide le long de l'axe longitudinal ; et
une commande coulissante d'électrode (134) couplée en service à l'électrode et configurée pour déplacer l'électrode le long de l'axe longitudinal,
où la canule à vide et l'électrode sont positionnables de manière indépendante et sélective par rapport au boîtier et l'une par rapport à l'autre en utilisant la commande coulissante de canule (136) et la commande coulissante d'électrode (134).

2. Instrument de dissection électrochirurgical selon la revendication 1, comprenant en outre un tube d'aspiration (120) ayant une extrémité proximale et une extrémité distale (121), configuré pour se coupler en service à la au moins une canule à vide (132).

3. Instrument de dissection électrochirurgical selon la revendication 2, dans lequel le tube d'aspiration (120) définit au moins un orifice de pré-aspiration (122) formé à proximité de l'extrémité distale de celui-ci.

4. Instrument de dissection électrochirurgical selon la revendication 2 ou 3, dans lequel le tube d'aspiration (120) est transparent.

5. Instrument de dissection électrochirurgical selon la revendication 2, 3 ou 4, dans lequel une surface du tube d'aspiration (120) inclut un revêtement choisi dans le groupe constitué d'un revêtement hydrophobe, d'un revêtement oléophobe et d'un revêtement lubrifiant.

6. Instrument de dissection électrochirurgical selon la revendication 2, 3, 4 ou 5, comprenant en outre un embout d'aspiration (310) disposé à une extrémité distale du tube d'aspiration (320), dans lequel l'embout d'aspiration est formé d'un matériau choisi dans le groupe constitué d'un matériau élastomère et d'un matériau rigide.

7. Instrument de dissection électrochirurgical selon l'une quelconque des revendications précédentes, comprenant en outre :
un générateur électrochirurgical (140) ; et
une source de vide (150),
dans lequel la au moins une canule à vide (132) est couplée en service à la source de vide et l'électrode (135) est couplée en service au générateur électrochirurgical.

8. Instrument de dissection électrochirurgical selon la revendication 7, comprenant en outre un actionneur à vide (131) disposé sur une surface externe du boîtier et configuré pour activer la source de vide.

9. Instrument de dissection électrochirurgical selon la revendication 7 ou 8, comprenant en outre un actionneur électrochirurgical (130) disposé sur une surface externe du boîtier et configuré pour activer le générateur électrochirurgical.

10. Instrument de dissection électrochirurgical selon la revendication 1, dans lequel la au moins une canule à vide inclut une première canule à vide (420) et une seconde canule à vide (425) disposée coaxialement dans la première canule à vide et la première et la seconde canules s'étendent de manière distale depuis le boîtier (415) et sont sélectivement mobiles l'une par rapport à l'autre et par rapport au boîtier (415) le long de l'axe longitudinal.
